(19) Europäisches Patentamt
European Patent Office
Office européen des brevets

(11) Publication number: 0 229 467 A1

(12) **EUROPEAN PATENT APPLICATION**

(21) Application number: 86309010.6

(22) Date of filing: 18.11.86

(51) Int. Cl.⁴: C07D 327/02 , A61K 31/39 , A61K 31/495

(30) Priority: 10.12.85 US 806809

(43) Date of publication of application: 22.07.87 Bulletin 87/30

(84) Designated Contracting States: AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Sugihara, Hirosada
5-604, 2 Minase 2-chome Shimamoto-cho
Mishima-gun Osaka 618(JP)
Inventor: Hirata, Minoru
26-13, Fushiodai 1-chome
Ikeda Osaka 563(JP)

(74) Representative: Laredo, Jack Joseph et al
Elkington and Fife High Holborn House 52/54
High Holborn
London, WC1V 6SH(GB)

(54) **1,5-Benzoxathiepin derivatives, their production and use.**

(57) Compounds of the formula:

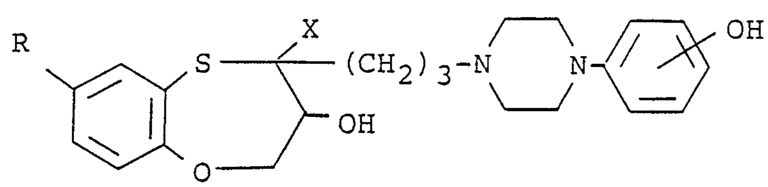

wherein R is hydroxy or lower alkoxy and X is lower alkoxycarbonyl, and their salts possess serotonin $S_2$ receptor blocking activity, calcium antagonism, actions to relieve cerebral vasospasm and to improve renal circulation, and diuretic and antithrombotic activities, and are useful as prophylactic and therapeutic agents for ischemic cardiopathies, thrombosis, hypertension and cerebral circulatory disorders.

EP 0 229 467 A1

# 1,5-Benzoxathiepin Derivatives, Their Production and Use

## Technological Field

The present invention relates to 1,5-benzoxathiepin derivatives useful as pharmaceuticals.

## Background Art

The present inventors have succeeded in producing 1,5-benzoxathiepin derivatives which exhibit serotonin $S_2$ receptor blocking activity, calcium antagonism, actions to relieve cerebral vasospasm and to improve renal circulation, and diuretic and antithrombotic activities, and are useful as prophylactic and therapeutic agents for ischemic cardiopathies such as angina pectoris and myocardial infarction, thrombosis, hypertension, and cerebral circulatory disorders such as cerebral vasospasm and transient ischemic attack (e.g. European Patent Publication of Application, Publication No. 0145494).

There are disclosed compounds of the following formula as preferred embodiments in the published specification:

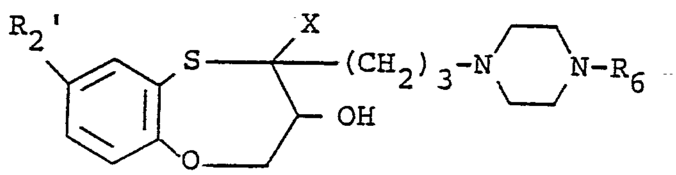

(I')

wherein $R_6$ is phenyl which may be substituted by 1 to 3 members of halogen, $C_{1-4}$ alkyl, $C_{1-4}$ alkoxy, methylenedioxy, amino, nitro or hydroxy, $R_2'$ is $C_{1-4}$alkoxy, and X is $C_{1-4}$ alkoxycarbonyl. However, no physico-chemical properties of compounds of the formula (I') wherein $R_6$ is hydroxy-substituted phenyl.

The present inventors, after further intensive research, have found that compounds of the formula (I') wherein $R_6$ is hydroxy-substituted phenyl have more excellent $S_2$ receptor blocking activity and completed the present invention.

## Disclosure of the Invention

The present invention provides compounds of the formula:

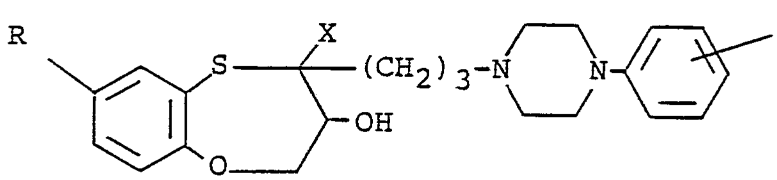

(I)

wherein R is hydroxy or lower alkoxy and X is lower alkoxycarbonyl, and their salts.

Referring to the above formula (I), the lower alkoxy group represented by R is exemplified by alkoxy groups containing 1 to 4 carbon atoms, such as methoxy, ethoxy, propoxy, isopropoxy, butoxy, isobutoxy, sec-butoxy and tert-butoxy. Referring to R, the methoxy group is the most preferable.

The lower alkoxycarbonyl group represented by X is exemplified by $C_{1-4}$ alkoxycarbonyl groups such as methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl. Referring to X, the methoxycarbonyl group is the most preferable.

The hydroxy group of hydroxyphenylpiperazinyl group may be attached to any position (ortho, meta, para) of phenyl group, but the para-position of the phenyl group is preferred.

Among the compounds (I), compounds of the formula (I) wherein the group

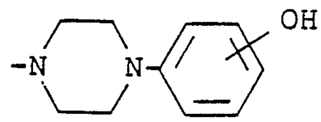

is 4-(4-hydroxyphenyl)piperazin-1-yl and R is lower alkoxy are more preferable. Among others, methyl cis-3-hydroxy-4-[3-[4-(4-hydroxyphenyl)piperazin-1-yl]-propyl]-7-methoxy-3,4-dihydro-2H-1,5-benzoxathiepin-4-carboxylate and its acid addition salt are the most preferable.

The salts of the compounds (I) are exemplified by pharmaceutically acceptable salts such as salts with inorganic acids (e.g. hydrochloride, hydrobromide, sulfate, nitrate, phosphate) and salts with organic acids - (e.g. acetate, tartrate, citrate, fumarate, maleate, toluenesulfonate, methanesulfonate).

The compound (I) of the present invention can be produced, for example, by reacting a compound of the formula:

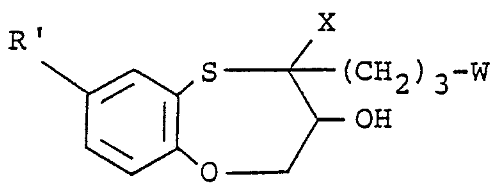

(II)

wherein W is halogen (e.g. bromine, chlorine) or a group represented by the formula: R″-$SO_2$-O-[wherein R″ is lower ($C_{1-4}$)alkyl, phenyl or p-tolyl], R′ is a lower ($C_{1-4}$)alkoxy group or hydroxy group which may be protected and X is as defined hereinbefore, with an amine of the formula:

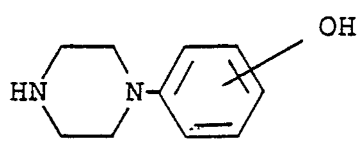

(III)

and, when R′ is a protected hydroxy group, by subjecting the obtained compound of the formula (I) wherein R is a protected hydroxy group to a deprotection reaction.

The reaction of the compound (II) with the amine (III) can be carried out in a suitable organic solvent - (e.g. methanol, ethanol, dioxane, acetonitrile, tetrahydrofuran, N,N-dimethylformamide, N,N-dimethylacetamide, methylene chloride, dimethylsulfoxide, and an arbitrary solvent mixture thereof). The reaction temperature is preferably in the range of 0°C to +150°C, and for the purpose of increasing the reaction rate, an organic base such as triethylamine, pyridine or N,N-dimethylaniline, or an inorganic base such as potassium carbonate, sodium carbonate or sodium hydrogencarbonate may be added as a catalyst.

The deprotection reaction to obtain a compound of the formula (I) wherein R is hydroxy by removing the protective group from the protected hydroxy group (e.g. benzyloxy, methoxymethyloxy) includes a catalytic reduction reaction using a metal such as platinum, palladium or rhodium, or a mixture thereof with an arbitrary support as a catalyst (when the protected hydroxy group is benzyloxy), and a hydrolysis reaction using an inorganic acid such as hydrochloric acid, sulfuric acid or phosphoric acid, or an organic acid such as formic acid or acetic acid as a catalyst (when the protected hydroxy group is methoxymethyloxy).

The above catalytic reduction reaction is normally carried out in the presence of water or an organic solvent (e.g. methanol, ethanol, ethyl ether, dioxane), and the reaction temperature varies with the means of reduction employed but generally is preferably in the range of -20°C to +100°C. This reaction can be conducted at atmospheric pressure to achieve the desired object satisfactorily, but may also be carried out under pressure or under reduced pressure according to the circumstances.

The above hydrolysis reaction is normally carried out in the presence of water or an organic solvent - (e.g. methanol, ethanol, dioxane, dichloromethane), and ordinarily in the temperature range of -20°C to +100°C.

The compound (I) of the present invention can also be produced, for example, by subjecting a compound of the formula:

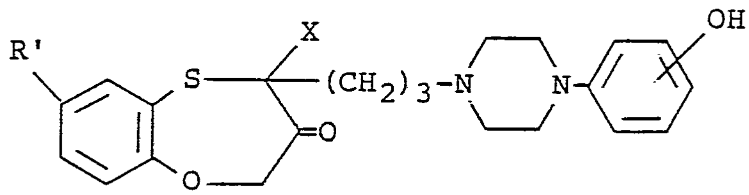

(IV)

wherein each of the symbols is as defined hereinbefore, to a reduction reaction, and, when R′ is a protected hydroxy group, by subjecting the obtained compound of the formula (I) wherein R is a protected hydroxy group to a deprotection reaction.

The reductive conditions include reduction with a metal hydride compound such as lithium alminum hydride, lithium borohydride, lithium cyanoborohydride, sodium borohyd ride, sodium cyanoborohydride or tri-tert-butoxylithium aluminum hydride; reduction with metallic sodium, metallic magnesium or the like and an alcohol; catalytic reduction using a metal such as platinum, palladium or rhodium, or a mixture thereof with an arbitrary support as a catalyst; reduction with a metal such as iron or zinc, and an acid such as hydrochloric acid or acetic acid; electrolytic reduction; reduction with a reducing enzyme; and reduction with a boron hydride compound such as diborane, or a complex of a boron hydride compound and an amine, such as borane-trimethylamine. The above reaction is normally carried out in the presence of water or an organic solvent (e.g. methanol, ethanol, ethyl ether, dioxane, methylene chloride, chloroform, benzene, toluene, acetic acid, dimethylformamide, dimethylacetamide), and the reaction temperature varies with the reduction means employed, but generally is preferably in the range of -20°C to +100°C.

The salts of the compounds (I), in some instances, can be obtained by the above-mentioned processes for producing the compounds (I) themselves, but can also be produced by adding an inorganic acid or an organic acid, if desired.

Referring to the compound (I), there exist at least four kinds of stereoisomers. These individual isomers and a mixture thereof, naturally, both fall within the scope of the present invention, and such isomers can also be produced individually, if desired. For example, a single isomer of the compound (I) can be produced by carrying out the above reaction using a single isomer of the starting compound (II). When the product is a mixture of not less than two kinds of isomers, it can be separated into individual isomers by a usual separation technique such as a method of forming a salt with an optically active acid (e.g. camphorsulfonic acid, tartaric acid, dibenzoyltartaric acid, malic acid), a variety of chromatographic techniques or fractional recrystallization.

The starting compounds (II) and (IV) can be produced, for example, by the methods as shown in the following reaction schema. Each of the intermediates can be obtained by a method similar to that as mentioned above or that described in European Patent Publication of Application, Publication No. 0145494.

Compound (II):

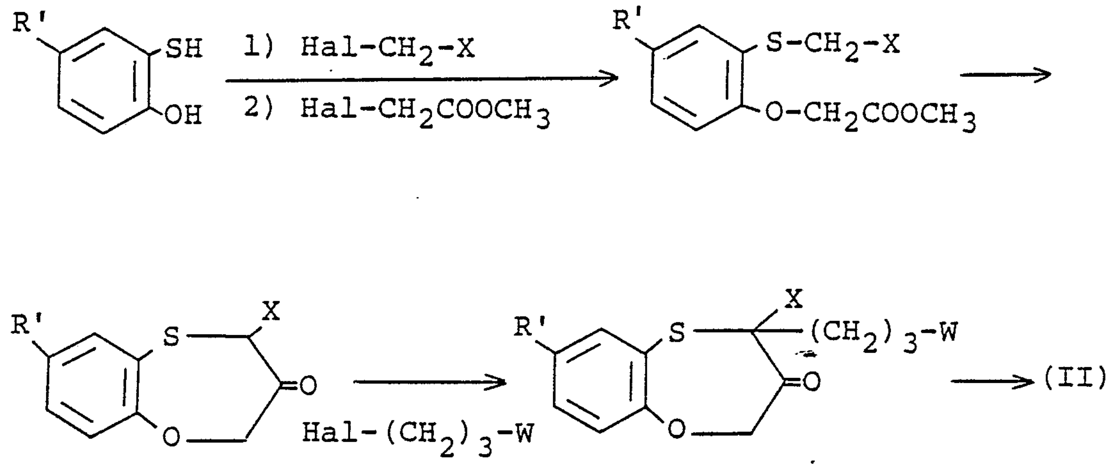


Compound (IV):

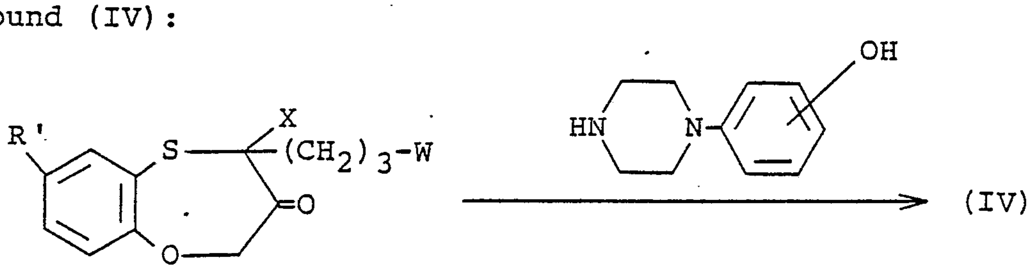


Hal: halogen (e.g. bromine, chlorine)

In the above processes for producing the compound (I) and intermediates thereof, the compounds which are used in the reactions may be used in the form of salts, such as inorganic acid salts being exemplified by hydrochloride, hydrobromide, sulfate, nitrate and phosphate; organic acid salts being exemplified by acetate, tartrate, citrate, fumarate, maleate, toluenesulfonate and methanesulfonate; metal salts being exemplified by sodium salt, potassium salt, calcium salt and aluminum salt; and salts with bases being exemplified by triethylamine salt, guanidine salt, ammonium salt, hydrazine salt, quinine salt and cinchonine salt, so long as they do not interfere with such reactions.

The compounds of the present invention, namely the 1,5-benzoxathiepin derivatives represented by the formula (I), exhibit specific serotonin $S_2$ receptor blocking activity, calcium antagonism, actions to relieve cerebral vasospasm and to improve renal circulation, diuretic and antithrombotic activities in animals, in particular, mammals (e.g., human, pigs, dogs, cats, rabbits, guinea pigs, rats, etc.), and are useful, for example, as drugs for prevention and treatment of ischemic cardiopathies, such as angina pectoris and myocardial infarction, thrombosis, hypertension and cerebral circulatory disorders, such as cerebral vasospasm and transient ischemic attack. The compounds of the present invention are of low toxicity, well absorbed even on oral administration and highly stable, and when they are used as the above-mentioned drugs, therefore, they can be safely administered orally or parenterally, per se or in admixture with suitable, pharmaceutically acceptable carriers, excipients or diluents in various pharmaceutical formulations, such as powders, granules, tablets, capsules and injectable solutions. While the dosage level varies depending upon the conditions of the diseases to be treated as well as the administration route, in the case of administration to human adult for the purpose of treatment of ischemic cardiopathies or hypertension, for example, the compounds (I) may be desirably administered orally at a single dose of, normally about 0.1 to 10 mg/kg, preferably about 0.3 to 3 mg/kg, or intravenously at a single dose of about 0.003 to 0.1 mg/kg, preferably about 0.01 to 0.1 mg/kg, about once to 3 times daily according to the conditions.

In the case of administration to a human adult for the purpose of treatment of cerebral circulatory disorders, for example, the compounds (I) may be desirably administered orally at a single dose of, normally about 0.1 to 50 mg/kg, preferably about 0.3 to 30 mg/kg, or intravenously at a single dose of about 0.003 to 10 mg/kg, preferably about 0.01 to I mg/kg, about once to 3 times per day according to the conditions.

The following Reference Examples, Examples, Experiment Example and Preparation Examples illustrate the present invention in more detail, but the present invention should not be limited thereto.

Each of the terms "cis" and "trans" shows relative configuration between the hydroxy group at the 3rd position of the 1,5-benzoxathiepin moiety and the alkoxycarbonyl group at the 4th position.

Reference Example 1

In 500 ml of acetone is dissolved 42 g of 5-benzyloxy-2-hydroxythiophenol, and 60 g of potassium carbonate is added. With stirring at room temperature, 65.4 g of methyl bromoacetate is added dropwise to the mixture over a period of 30 minutes, and the mixture is stirred at room temperature for 2 hours, followed by heating under reflux for 4 hours. The inorganic material is filtered off and the filtrate is concentrated under reduced pressure. The residue is purified by column chromatography on silica gel (eluent: hexane: ethyl acetate = 1:1) to give 42 g of crystals of methyl 4-benzyloxy-2-methoxycarbonylmethylthiophenoxyacetate. Recrystallization from methanol gives colorless prisms.

Melting point: 52-53°C
Elemental Analysis for $C_{19}H_{20}O_6S$
Calcd.: C, 60.63; H, 5.36
Found : C, 60.75; H, 5.39

Example 2

In 150 ml of N,N-dimethylformamide is dissolved 42 g of methyl 4-benzyloxy-2-methoxycarbonylmethylthiophenoxyacetate, and 23.7 g of 28% sodium methoxide (in methanol) in 50 ml of N,N-dimethylformamide is added dropwise to the mixture over a period of 30 minutes with stirring under ice-cooling. The resulting mixture is stirred at room temperature for 1 hour, and then poured into ice-water containing 8 g of acetic acid, followed by extraction with ethyl acetate. The ethyl acetate layer is washed with water, dried and concentrated under reduced pressure. The residue is crystallized from isopropyl ether to give 33.2 g of methyl 7-benzyloxy-3-oxo-3,4-dihydro-2H-1,5-benzoxathiepin-4-carboxylate.

Melting point: 101-102°C
Elemental Analysis for $C_{18}H_{16}O_5S$
Calcd.: C, 62.78; H, 4.68
Found : C, 62.71; H, 4.38

Reference Example 3

A mixture of 33.2 g of methyl 7-benzyloxy-3-oxo-3,4-dihydro-2H-1,5-benzoxathiepin-4-carboxylate, 40 g of 1-bromo-3-chloropropane, 30 g of potassium carbonate, 8 g of potassium iodide and 400 ml of acetonitrile is heated under reflux with stirring for 6 hours. The mixture is filtered and the filtrate is concentrated under reduced pressure. The residue is purified by column chromatography on silica gel (eluent: hexane: ethyl acetate = 2:1) to give 30 g of a colorless oil of methyl 7-benzyloxy-4-(3-chloropropyl)-3-oxo-3,4-dihydro-2H-1,5-benzoxathiepin-4-carboxylate.

IR $\nu_{max}^{neat}$ cm$^{-1}$: 1755, 1725
NMR($CDCl_3$)δ: 3.72 (3H, singlet), 4.60 (2H, double doublet)

Reference Example 4

In a mixture of 30 ml of tetrahydrofuran and 150 ml of methanol is dissolved 30 g of methyl 7-benzyloxy-4-(3-chloropropyl)-3-oxo-3,4-dihydro-2H-1,5-benzoxathiepin-4-carboxylate, and 2.0 g of sodium borohydride is added with stirring under ice-cooling. The mixture is concentrated under reduced pressure, and then the residue is diluted with water and extracted with ethyl acetate. The ethyl acetate layer is washed with water, dried and concentrated under reduced pressure. The residue is purified by column chromatography on silica gel (eluent: hexane: ethyl acetate = 3:1) to obtain from the first fraction 9.1 g of crystals of methyl trans-7-benzyloxy-4-(3-chloropropyl)-3-hydroxy-3,4-dihydro-2H-1,5-benzoxathiepin-4-carboxylate. Recrystallization from ethyl acetate and hexane gives colorless prisms.

Melting point: 97-99°C

Elemental Analysis for $C_{21}H_{23}ClO_5S$

Calcd.: C, 59.64; H, 5.48

Found : C, 59.80; H, 5.51

From the subsequent fraction eluted, 15.2 g of methyl cis-7-benzyloxy-4-(3-chloropropyl)-3-hydroxy-3,4-dihydro-2H-1,5-benzoxathiepin-4-carboxylate is obtained as a colorless oil.

IR $\nu_{max}^{neat}$ $cm^{-1}$: 13550 (OH), 1740 (ester)

Elemental Analysis for $C_{21}H_{23}ClO_5S$

Calcd.: C, 59.64; H, 5.48

Found : C, 59.77; H, 5.39

Reference Example 5

A mixture of 3.0 g of methyl cis-7-benzyloxy-4-(3-chloropropyl)-3-hydroxy-3,4-dihydro-2H-1,5-benzoxathiepin-4-carboxylate, 1.9 g of N-4-hydroxyphenylpiperazine and 5 ml of N,N-dimethylacetamide is stirred at 90°C for 6 hours. Water is added to the mixture and the mixture is extracted with ethyl acetate. The organic layers are combined, washed with water and dried and the solvent is evaporated off under reduced pressure. The residue is purified by silica gel column chromatography [eluent: hexane-ethyl acetate-methanol (70:150:6)] to give 2.3 g of methyl cis-7-benzyloxy-3]-hydroxy-4-[3-[4-(4-hydroxyphenyl)-piperazin-1-yl]propyl]-3,4-dihydro-2H-1,5-benzoxathiepin-4-carboxylate as a pale yellow oil. The product is converted to a powder of the hydrochloride.

Elemental Analysis for $C_{31}H_{36}N_2O_6S \bullet HCl$

Calcd.: C, 61.93; H, 6.20; N, 4.66

Found : C, 61.91; H, 6.01; N, 4.65

Example 1

A mixture of 1.0 g of methyl cis-4-(3-chloropropyl)-3-hydroxy-7-methoxy-3,4-dihydro-2H-1,5-benzoxathiepin-4-carboxylate, 0.67 g of N-4-hydroxyphenylpiperazine and 2 ml of N,N-dimethyl-acetamide is stirred at 90°C for 3 hours. Water is added to the mixture and the mixture is extracted with ethyl acetate. The organic layers are combined, washed with water and dried and the solvent is evaporated off under reduced pressure. The residue is purified by silica gel column chromatography [eluent: hexane-ethyl acetate-methanol (20:20:1)] to give 0.7 g of methyl cis-3-hydroxy-4-[3-[4-(4-hydroxyphenyl)piperazin-1-yl]-propyl]-7-methoxy-3,4-dihydro-2H-1,5-benzoxathiepin-4-carboxylate as a pale yellow oil. This product is converted to a white powder of the dihydrochloride.

Melting point: 240-245°C (decomp.)

Elemental Analysis for $C_{25}H_{32}N_2O_6S \bullet 2HCl$

Calcd.: C, 53.47; H, 6.10; N, 4.99

Found : C, 53.20; H, 5.97; N, 5.21

Example 2

A mixture of 1.0 g methyl cis-4-(3-chloropropyl)-3-hydroxy-7-methoxy-3,4-dihydro-2H-1,5-benzoxathiepin-4-carboxylate, 1.2 g of N-2-hydroxyphenylpiperazine, 0.97 g of potassium carbonate and 6 ml of N,N-dimethylacetamide is stirred at 85°C for 5 hours. Water is added to the mixture and the mixture is extracted with ethyl acetate. The organic layers are combined, washed with water and dried and the solvent is evaporated off under reduced pressure. The residue is purified by silica gel column chromatography [eluent: hexane-ethyl acetate-methanol (20:20:1)] to give 0.7 g of methyl cis-3-hydroxy-4-[3-[4-(2-hydroxyphenyl)piperazin-1-yl]propyl]-7-methoxy-3,4-dihydro-2H-1,5-benzoxathiepin-4-carboxylate as a pale yellow oil. This product is converted to a white powder of the dihydrochloride.

Elemental Analysis for $C_{25}H_{32}N_2O_6S•2HCl•1/2H_2O$
Calcd.: C, 52.63; H, 6.18; N, 4.91
Found : C, 52.91; H, 5.94; N, 4.95

Example 3

In 600 ml of methanol is dissolved 1.16 g of methyl cis-7-benzyloxy-3-hydroxy-4-[3-[4-(4-hydroxyphenyl)piperazin-1-yl]propyl]-3,4-dihydro-2H-1,5-benzoxathiepin-4-carboxylate, and 1.0 g of 10% palladium carbon and 0.1 ml of concentrated hydrochloric acid are added to the mixture, followed by stirring at ambient temperature and under atmospheric pressure. The catalyst is filtered off and the solvent is evaporated off. The residue is purified by silica gel column chromatography [eluent: hexane-ethyl acetate-methanol (12:12:1)]. The obtained crude product is recrystallized from ethyl acetate to give 0.62 g of methyl cis-3,7-dihydroxy-4-[3-[4-(4-hydroxyphenyl)piperazin-1-yl]propyl]-3,4-dihydro-2H-1,5-benzoxathiepin-4-carboxylate as a white powder.

Melting point: 226-229°C
Elemental Analysis for $C_{24}H_{30}N_2O_6S$
Calcd.: C, 60.74; H, 6.37; N, 5.90
Found : C, 60.47; H, 6.39; N, 5.71

Preparation Examples

The compounds (I) of the present invention can be used, for example, as a therapeutic agent for ischemic cardiopathies, in the following examples of formulation.

1. Tablets

| | | |
|---|---|---|
| (1) | Methyl cis-3-hydroxy-4-[3-[4-(4-hydroxyphenyl)piperazin-1-yl]propyl]-7-methoxy-3,4-dihydro-2H-1,5-benzoxathiepin-4-carboxylate•dihydrochloride | 10 g |
| (2) | Lactose | 90 g |
| (3) | Corn starch | 29 g |
| (4) | Magnesium stearate | 1 g |
| | For 1000 tablets, | 130 g |

The above ingredients (1) and (2) and 17 g of (3) are blended, and granulated together with a paste prepared from 7 g of the ingredient (3). 5 g of the ingredient (3) and the ingredient (4) are added to the resulting granules, and the mixture is compressed by a tabletting machine to prepare 1000 tablets of diameter of 7 mm each containing 10 mg of the ingredient (1).

2. Capsules

| | | |
|---|---|---|
| (1) | Methyl cis-3-hydroxy-4-[3-[4-(4-hydroxyphenyl)piperazin-1-yl]propyl]-7-methoxy-3,4-dihydro-2H-1,5-benzoxathiepin-4-carboxylate• dihydrochloride | 10 g |
| (2) | Lactose | 135 g |
| (3) | Finely powdered cellulose | 70 g |
| (4) | Magnesium stearate | 5 g |
| | For 1000 capsules, | 220 g |

All of the above ingredients are blended and filled into 1000 capsules of Gelatin Capsule No. 3 (X Japanese Pharmacopoeia) to prepare 1000 capsules each containing 10 mg of the ingredient (1).

3. Injectable solution

(1) Methyl cis-3-hydroxy-4-[3-[4-(4-hydroxy phenyl)piperazin-1-yl]propyl]-7-methoxy-3,4-dihydro-2H-1,5-benzoxathiepin-4-carboxylate. dihydrochloride 10 g

(2) Sodium chloride 9 g

(3) Chlorobutanol 5 g

All of the ingredients are dissolved in 1000 ml of distilled water, and charged into 1000 brown ampoules each containing 1 ml of the solution. The air in the ampoules is replaced with nitrogen gas and the ampoules are sealed. The preparation steps are conducted entirely under sterile conditions.

Experiment Example 1

Serotonin $S_2$-receptor blocking activity (in vitro) of the compound of the present invention:

[Experimental method]

The experiment was carried out in accordance with the method of Bevan & Osher (Agents Actions, 2, 257, 1972) with a few modifications. The heart removed from a hog immediately after being slaughtered at a slaughterhouse was preserved under ice-cooling, and the left circumflex coronary artery was dissected within 3 hours. The coronary artery was cut into a ring preparation of about 3 mm in width, which was suspended in a double-wall organ bath containing 20 ml of the Krebs-Henseleit solution using a pair of suspending hooks. One of the suspending hooks was fixed to the bottom of the organ bath, while the other was connected to a strain-gauge transducer, and the constriction of the ring preparation of the porcine coronary artery was isometrically measured and recorded on a polygraph recorder. The organ bath was maintained at 37°C, and the Krebs-Henseleit solution was saturated with a mixed gas of 97% $O_2$ + 3% $CO_2$, with the Krebs-Henseleit solution being composed of 118.3 mM NaCl, 4.7 mM KCl, 1.2 mM $KH_2PO_4$, 2.58 mM $CaCl_2 \cdot 2H_2O$, 1.15 mM $MgSO_4 \cdot 7H_2O$, 25 mM $NaHCO_3$ and 11.1 mM glucose.

In 1 to 2 hours when the blood vessel preparation showed a stable resting tension, the resting tension was readjusted to be 2 g, and $10^{-5}$M serotonin (final concentration) was added to the organ bath at intervals of about 1 hour to check the responsiveness of the preparation. When the reaction of the blood vessel to 2 or 3 additions of serotonin became stable, a concentration of the test compound was added to the organ bath 10 minutes prior to subsequent addition of serotonin. The serotonergic blocking effect of the test compound was calculated from the magnitudes of constriction caused by serotonin before and after the addition of the test compound.

[Experimental results]

The results of the experiment with regard to the compounds of the present invention are shown in Table 1.

Table 1:

Serotonin $S_2$-receptor blocking effect in porcine coronary artery preparation

| Example No. | Concn. (M) | Inhibition of constriction by serotonin (%) |
|---|---|---|
| 1 | $10^{-5}$ | 100 |
| | $10^{-6}$ | 100 |
| | $10^{-7}$ | 62 |
| 2 | $10^{-5}$ | 100 |
| 3 | $10^{-5}$ | 100 |

**Claims**

1. A compound of the formula (I):

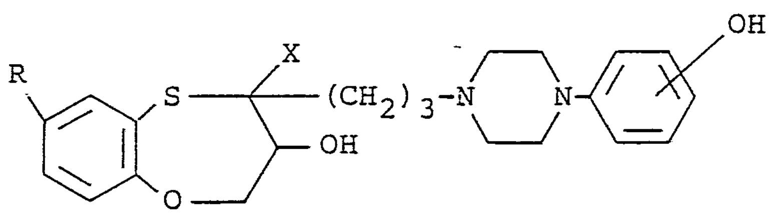

(I)

wherein R is hydroxy or $C_{1-4}$ alkoxy and X is $C_{1-4}$alkoxycarbonyl, or a pharmaceutically acceptable salt thereof.

2. A compound according to claim 1, wherein the group:

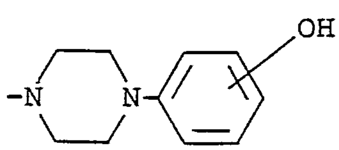

is 4-(4-hydroxyphenyl)piperazin-1-yl.

3. A compound according to claim 1, wherein X is methoxycarbonyl.

4. A compound according to claim l, wherein R is $C_{1-4}$ alkoxy.

5. A compound according to claim 1, wherein R is methoxy.

6. A compound according to claim 1, which is methyl cis-3-hydroxy-4-[3-[4-(4-hydroxyphenyl)piperazin-1-yl]propyl]-7-methoxy-3,4-dihydro-2H-1,5-benzoxathiepin-4-carboxylate or a pharmaceutically acceptable acid addition salt thereof.

7. A method for producing a compound of the formula (I):

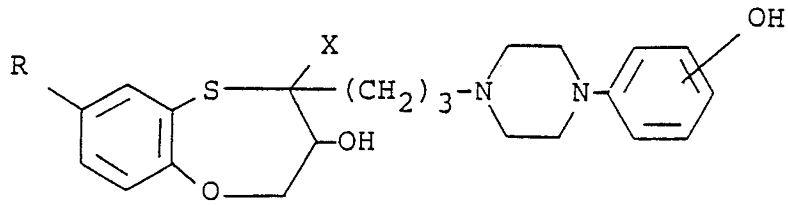

(I)

wherein R is hydroxy or C $_{1-4}$ alkoxy and X is $C_{1-4}$alkoxycarbonyl, or a pharmaceutically acceptable salt thereof, which comprises

(a)reacting a compound of the formula (II):

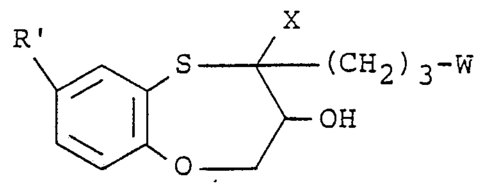

(II)

wherein W is halogen or a group of the formula: R″-$SO_2$-O-wherein R″ is $C_{1-4}$ alkyl, phenyl or p-tolyl; R′ is $C_{1-4}$ alkoxy or hydroxy group which may be protected; and X is as defined hereinbefore, with an amine of the formula (III):

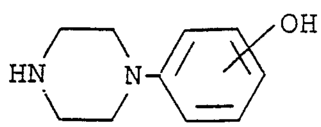

(III)

or (b) subjecting a compound of the formula (IV):

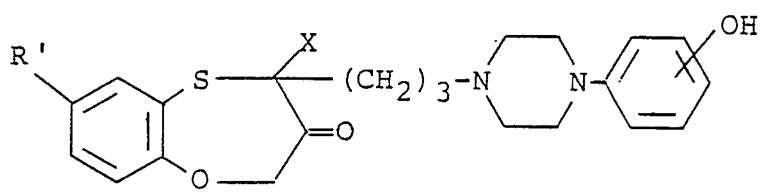

(IV)

wherein each of the symbols is as defined hereinbefore, to a reduction reaction,

and if desired, (c) subjecting the obtained compound of the formula (I) wherein R is a protected hydroxy group, to a deprotection reaction, to give a compound of the formula (I) wherein R is hydroxy,

and if desired, (d) converting the obtained compound of the formula (I) into a pharmaceutically acceptable salt thereof.

8. A method according to claim 7, wherein the starting compound is a compound of the formula (II) or -(IV) wherein X is methoxycarbonyl.

9. A method according to claim 7, wherein the starting compound is a compound of the formula (II) or -(IV) wherein R′ is $C_{1-4}$ alkoxy.

10. A method according to claim 7, wherein the starting compound is a compound of the formula (II) or (IV) wherein R′ is methoxy.

11. A method according to claim 7, wherein the starting compound is a compound of the formula (III) or (IV) wherein the group

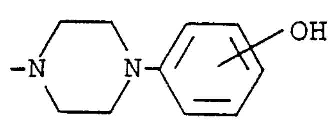

is 4-(4-hydroxyphenyl)piperazin-1-yl.

12. A method according to claim 7, for producing methyl cis-3-hydroxy-4-[3-[4-(4-hydroxyphenyl)-piperazin-1-yl]propyl]-7-methoxy-3,4-dihydro-2H-1,5-benzoxathiepin-4-carboxylate or a pharmaceutically acceptable acid addition salt thereof, which comprises reacting methyl cis-4-(3-halopropyl)-3-hydroxy-7-methoxy-3,4-dihydro-2H-1,5-benzoxathiepin-4-carboxylate with N-4-hydroxyphenylpiperazine, and if desired, converting the desired product to a pharmaceutically acceptable acid addition salt thereof.

13. A method according to claim 7, wherein the starting compound is a compound of the formula (II) wherein W is halogen.

14. A pharmaceutical composition which comprises, as an active ingredient, an effective amount of a compound as claimed in any one of claims 1 to 6 in association with a pharmaceutically acceptable carrier, excipient or diluent therefor.

15. The use of a compound as claimed in any one of claims 1 to 6 or a pharmaceutical composition as claimed in claim 14 for the manufacture of a medicament with serotin $S_2$ receptor blocking activity, calcium antagonism, cerebral vasospasm relieving activity, renal circulation improving activity, diurectic or antithrombotic activities, for prophylactic and therapeutic agents for ischemic cardiopathics, thrombosis, hypertension and cerebral circulatory disorders.

Claims for the following Contracting States : AT ; ES; and GR

1. A method for producing a compound of the formula (I):

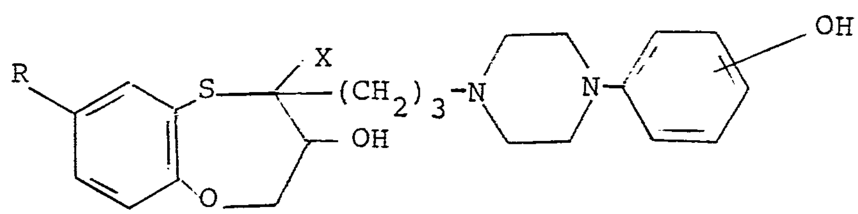

(I)

wherein R is hydroxy or $C_{1-4}$ alkoxy and X is $C_{1-4}$alkoxycarbonyl, or a pharmaceutically acceptable salt thereof, which comprises

(a) reacting a compound of the formula (II):

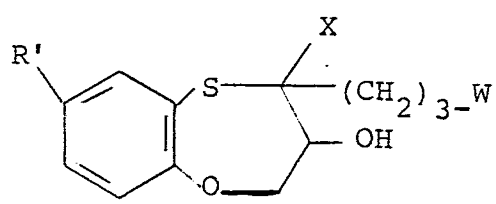

(II)

wherein W is halogen or a group of the formula: R"-$SO_2$-O-wherein R" is $C_{1-4}$ alkyl, phenyl or p-tolyl; R' is $C_{1-4}$ alkoxy or hydroxy group which may be protected; and X is as defined hereinbefore, with an amine of the formula (III):

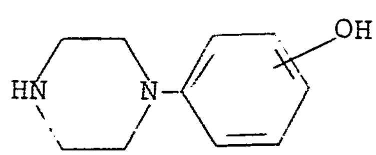

(III)

or (b) subjecting a compound of the formula (IV):

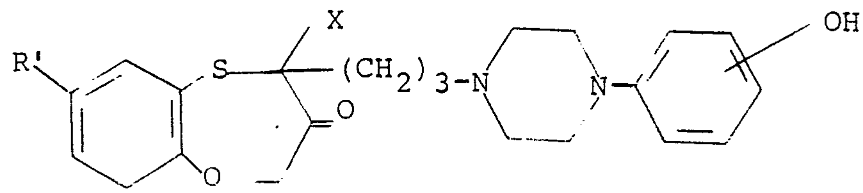

(IV)

wherein each of the symbols is as defined hereinbefore, to a reduction reaction,

and if desired, (c) subjecting the obtained compound of the formula (I) wherein R is a protected hydroxy group, to a deprotection reaction, to give a compound of the formula (I) wherein R is hydroxy,

and if desired, (d) converting the obtained compound of the formula (I) into a pharmaceutically acceptable salt thereof.

2. A method according to claim I, wherein the starting compound is a compound of the formula (II) or (IV) wherein X is methoxycarbonyl.

3. A method according to claim I, wherein the starting compound is a compound of the formula (II) or (IV) wherein R′ is $C_{1-4}$ alkoxy.

4. A method according to claim I, wherein the starting compound is a compound of the formula (II) or (IV) wherein R′ is methoxy.

5. A method according to claim I, wherein the starting compound is a compound of the formula (III) or (IV) wherein the group

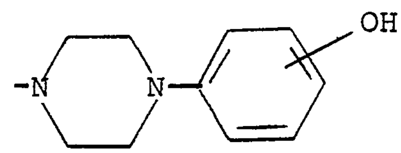

is 4-(4-hydroxyphenyl)piperazin-I-yl.

6. A method according to claim I, for producing methyl cis-3-hydroxy-4-[3-[4-(4-hydroxyphenyl)-piperazin-I-yl]propyl]-7-methoxy-3,4-dihydro-2H-I,5-benzoxathiepin-4-carboxylate or a pharmaceutically acceptable acid addition salt thereof, which comprises reacting methyl cis-4-(3-halopropyl)-3-hydroxy-7-methoxy-3,4-dihydro-2H-I,5-benzoxathiepin-4-carboxylate with N-4-hydroxyphenylpiperazine, and if desired, converting the desired product to a pharmaceutically acceptable acid addition salt thereof.

7. A method according to claim I, wherein the starting compound is a compound of the formula (II) wherein W is halogen.

8. A pharmaceutical composition which comprises, as an active ingredient, an effective amount of a compound(I) as defined in any one of claims I to 7 in association with a pharmaceutically acceptable carrier, excipient or diluent therefor.

9. The use of a compound (I) as defined in any one of claims I to 7 or a pharmaceutical composition as claimed in claim 8 for the manufacture of a medicament with serotin $S_2$ receptor blocking activity, calcium antogonism, cerebral vasospasm relieving activity, renal circulation improving activity, diurectic or antithrombotic activities, for prophylactic and therapeutic agents for ischemic cardiopathics, thrombosis, hypertension and cerebral circulatory disorders.

European Patent Office

# EUROPEAN SEARCH REPORT

Application number

EP 86309010.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP - A2/A3 - 0 145 494 (TAKEDA CHEMICAL INDUSTRIES)<br>* Claims 48-49; claim 30 *<br>---- | 1-5,7-11,14,15 | C 07 D 327/02<br>A 61 K 31/39<br>A 61 K 31/495 |
| | | | TECHNICAL FIELDS SEARCHED (Int. Cl.4) |
| | | | C 07 D 327/00 |

The present search report has been drawn up for all claims

| Place of search | Date of completion of the search | Examiner |
|---|---|---|
| VIENNA | 09-03-1987 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document